# EUROPEAN PATENT SPECIFICATION

(11) **EP 3 988 142 B1**
(45) Date of publication and mention of the grant of the patent: **27.11.2024**
(21) Application number: 21202390.7
(22) Date of filing: 13.10.2021
(51) Int. Cl.: A61M 1/36, A61M 5/36, G01N 29/02

(54) **BUBBLE DETECTOR**
BLASENDETEKTOR
DÉTECTEUR DE BULLES

(30) Priority: 22.10.2020 JP 2020177096
(43) Date of publication of application: 27.04.2022
(73) Proprietor: Shibuya Corporation, Kanazawa-shi, Ishikawa 920-8681 (JP)
(72) Inventor: NAKAGAWA, Ryota, Ishikawa, 920-8681 (JP)
(74) Representative: Mewburn Ellis LLP

(56) References cited:
- US-A1- 2008 011 060
- US-A1- 2008 098 798
- US-A1- 2013 180 341

## Description

### BACKGROUND OF THE INVENTION

### Field of the Invention

The present invention relates to a bubble detector for detecting existence of bubbles in a liquid flowing through a tube.

### Description of the Related Art

A bubble detector of generally known type includes a first ultrasonic element disposed on a first support member, a second ultrasonic element disposed on a second support member to face the first ultrasonic element with a tube intervening therebetween, and a first distance adjuster for adjusting the distance between the first support member and the second support member so that the distance between the first ultrasonic element and the second ultrasonic element is adjusted in accordance with the diameter of the tube. Ultrasonic waves transmitted from one of the first ultrasonic element and the second ultrasonic element are received by the other ultrasonic element so that existence of bubbles in a liquid flowing through the tube can be detected (patent literature 1).

The transmission efficiency of the ultrasonic waves in the liquid is different from that of the ultrasonic waves in the bubble. In the presence of bubbles in the liquid, the ultrasonic waves are blocked by the bubbles, which weakens receiving intensity. The bubbles can be detected using this feature. It is necessary to bring the first and the second ultrasonic elements into tight contact with the tube to accurately detect the bubbles. When a small-diameter tube is used along with other tubes each having a different diameter, the first and the second ultrasonic elements cannot be brought into tight contact with such a tube appropriately unless the distance between the first and the second ultrasonic elements is made adjustable. This may result in deteriorated detection accuracy.

The above-described bubble detector is configured to allow the first distance adjuster to adjust the distance between the first and the second ultrasonic elements. Even when tubes of different types are used, the first and the second ultrasonic elements can be constantly brought into tight contact with the tubes appropriately. This makes it possible to prevent the deterioration of detection accuracy.

Patent Literature 2 discloses a universal air bubble detector for use with a variety of sizes and types of tubing. The detector maintains proper alignment of a sensor emitter and receiver with different sizes of tubing, by means of pivot arms or sliding mounts for adjusting the distance between a support carrying the emitter and a support carrying the receiver, as well as of guiding structures of said supports, disposed on either side of the emitter and receiver, respectively. The detector may be mounted on existing equipment or may be used to monitor a tubing at any position along the tubing and may operate in a stand-alone mode or in combination with existing equipment.

Patent Literature 3 discloses a conduit clamped within a clamping hole formed by a main body, left and right side plates and a top plate. The clamping hole is maintained by a locking mechanism and the conduit is deformed to have a substantially square cross section. Ultrasonic signal transmission and reception elements are arranged in the main body such that an ultrasonic beam emitted from one of the elements is received by the other element after the beam is reflected by the top plate.

Patent Literature 4 discloses an apparatus for determining a characteristic such as sound speed for a fluid such as blood in a tube, the apparatus comprising means for applying a force to cause a change in a transverse dimension of a segment of the tube, wherein the change in the transverse dimension is reversible, and means for launching and receiving ultrasonic signals. The applied force may cause the transverse dimension to change continuously or in discrete steps. The characteristic is determined based on information comprising the path length differences and the transit times for each of the ultrasonic signals.

### [Citation List]

### [Patent Literature]

Patent Literature 1: Japanese Patent No. 3447957
Patent Literature 2: US2008/098798
Patent Literature 3: US2013/180341
Patent Literature 4: US2008/011060

### SUMMARY OF THE INVENTION

### [Technical Problem]

The first distance adjuster of the generally employed bubble detector (patent literature 1) allows the first and the second ultrasonic elements to press the tube in the radial direction. Accordingly, the tube is elliptically pressed to reduce the flow passage area inside the tube. This may cause the risk of failing to secure a required flow rate.

In light of the circumstances, it is an object of the present invention to provide the bubble detector which allows the first and the second ultrasonic elements to be in tight contact with the tube appropriately, and prevents the tube from being pressed elliptically to secure a required flow rate.

### [Solution to Problem]

According to the present invention there is provided a bubble detector as specified in claim 1.

### [Advantageous Effects of Invention]

With the invention according to claim 1, since the distance between the first and the second ultrasonic elements can be adjusted by the first distance adjuster, even if the diameter of the tube is changed, the first and the second ultrasonic elements can be brought into tight contact with the tube appropriately like the generally employed case. This makes it possible to secure detection of bubbles.

At the same time, a pair of side guides facing with each other with the tube intervening therebetween are arranged in the direction intersecting the arrangement direction of the first and the second ultrasonic elements. Further, the second distance adjuster adjusts the distance between those side guides in accordance with the diameter of the tube. This prevents the tube from being elliptically pressed by the first and the second ultrasonic elements and allows the tube to be deformed rectangularly. It is therefore possible to secure a required flow rate by preventing reduction in the flow passage area of the inside of the tube.

### BRIEF DESCRIPTION OF THE DRAWINGS

FIG. 1 is a schematic circuit diagram of a blood purifier.
FIG. 2 is a perspective view of a detection box 11 assembled with a bubble detector 1 according to the present invention.
FIG. 3 is an exploded perspective view of the bubble detector 1 according to the present invention.
FIG. 4 is a sectional view of the bubble detector 1 in an initial state.
FIG. 5 is a sectional view of the bubble detector 1 which is adjusted adaptively to a large-diameter tube 19.
FIG. 6 is a sectional view of the bubble detector 1 which is adjusted adaptively to a small-diameter tube 19.

### DETAILED DESCRIPTION OF THE PREFERRED EMBODIMENTS

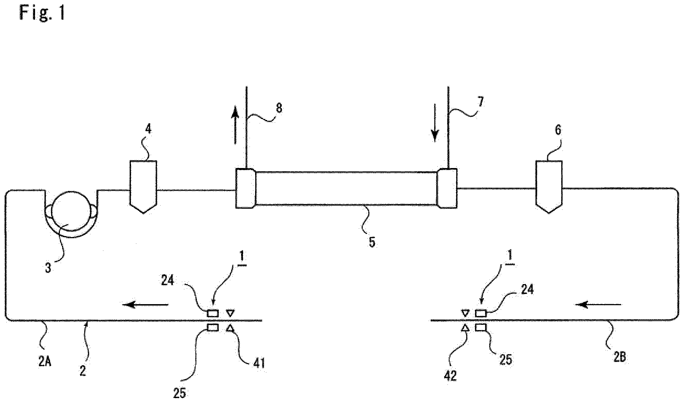

The present invention will be described based on an illustrated example. FIG. 1 is a schematic circuit diagram of a generally known blood purifier. Two bubble detectors 1 according to the present invention are incorporated in the blood purifier. Each of the detectors is configured to detect existence of bubbles in the blood flowing through a blood circuit 2.

The blood circuit 2 comprises an arterial-side blood circuit 2A and a venous-side blood circuit 2B. A non-illustrated puncture needle disposed at one end of the arterial-side blood circuit 2A is configured to be connected to a patient. The other end of the arterial-side blood circuit 2A is connected to one end of a dialyzer 5 via a blood pump 3 and an arterial-side chamber 4.

A terminal end of the venous-side blood circuit 2B is connected to the other end of the dialyzer 5. A top end of the venous-side blood circuit 2B is configured to be connected to the patient via a venous-side chamber 6 and a non-illustrated puncture needle.

The dialyzer 5 is configured to be supplied with fresh dialysis fluid from a dialysis fluid supply circuit 7. The dialysis fluid supplied into the inside of the dialyzer 5 is discharged to the outside via a dialysis fluid discharge circuit 8 connected to the dialyzer 5.

Although not illustrated, a large number of hollow fibers are provided in the dialyzer 5 in which the blood from the arterial-side blood circuit 2A flows to the venous-side blood circuit 2B via the hollow fibers.

Meanwhile, the dialysis fluid supplied into the dialyzer 5 circulates therein on the outer side of the hollow fibers so that the blood is separated from the dialysis fluid via the hollow fibers. Wastes in the blood flowing through the hollow fibers are drawn into the dialysis fluid flowing outside the hollow fibers so that the blood is purified.

As the blood purifier of the above-described type is known, further explanation of the blood purifier will be omitted.

One of the two bubble detectors 1, 1 according to the present invention is disposed on an inlet side of the arterial-side blood circuit 2A, and the other bubble detector 1 is disposed on an outlet side of the venous-side blood circuit 2B so that existence of bubbles in the blood flowing through the respective blood circuits 2A, 2B can be detected.

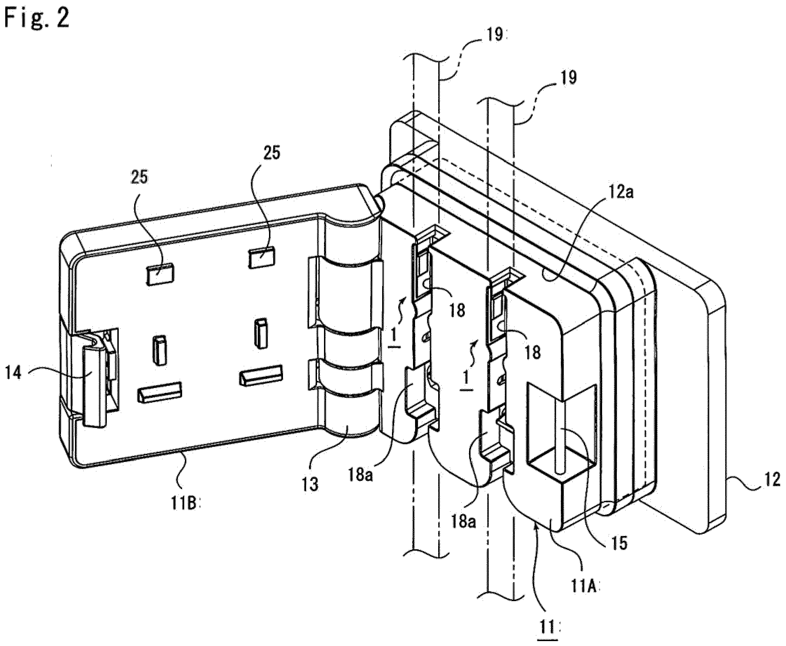

FIG. 2 is a perspective view of a detection box 11 assembled with the two bubble detectors 1, 1. The detection box 11 is detachably attached to a bracket 12 fixed to a front panel of a non-illustrated blood purifier. More specifically, the detection box 11 has a rectangular box shape, with its rear part detachably fittable with a rectangular recess part 12a formed in a front surface of the bracket 12. It is possible to provide a falling prevention member that prevents the detection box 11 from falling from the bracket 12 as needed.

The detection box 11 includes a rectangular box-like main body 11A and a cover 11B openably attached via a hinge 13 disposed on one side of the main body 11A. When the cover 11B is closed, a latch 14 disposed on the other side of the cover 11B is engaged with a locking pin 15 disposed on the other side of the main body 11A so that the cover 11B is kept closed.

Operating the latch 14 from the closed state so as to disengage the latch 14 from the locking pin 15 allows the cover 11B to be opened from the closed state as shown in FIG. 2.

The main body 11A of the detection box 11 has two tube grooves 18 that run parallel to each other in an up-down direction. A tube 19 that constitutes the arterial-side blood circuit 2A is allowed to be fitted with one of the tube grooves 18, and a tube 19 that constitutes the venous-side blood circuit 2B is allowed to be fitted with the other tube groove 18.

Each of the two bubble detectors 1 is configured to detect bubbles in the blood flowing through the tube 19 fitted with each of the tube grooves 18.

As the two bubble detectors 1 have the same structure, an explanation will be made only about one of those bubble detectors 1 in detail.

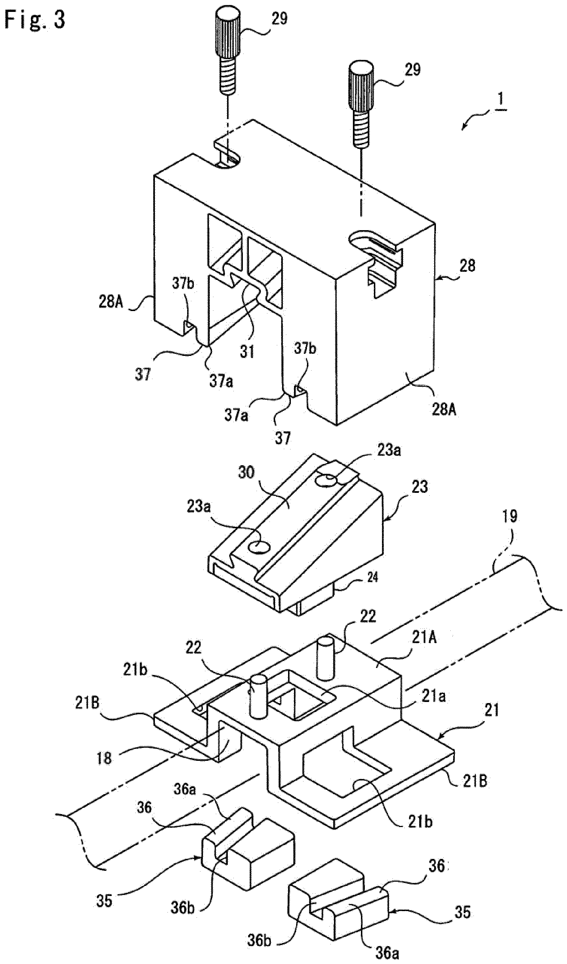

FIG. 3 is an exploded perspective view of the bubble detector 1 in a disassembled state. The bubble detector 1 includes a tube guide 21. The tube guide 21 has a U-like cross section part 21A and two base parts 21B each horizontally extending from the proximal end thereof. Both of the base parts 21B are fixed to a surface side (side that faces the cover 11B) of the main body 11A of the detection box 11. The inner surface of the U-like cross section part 21A constitutes a part of the tube groove 18.

Referring to FIG. 3, the tube groove 18 of the tube guide 21 is expressed as being laterally directed. In the state where the detection box 11 is assembled with the bracket 12 of the blood purifier, the tube groove 18 of the tube guide 21 is disposed in the up-down direction. In other words, the tube groove is disposed so that its left side shown in FIG. 3 is directed at the upper side.

Two guide pins 22 parallel to each other are disposed on the upper surface of the U-like cross section part 21A of the tube guide 21 at both ends in its longitudinal direction. Those guide pins 22 are engaged with two engagement holes 23a formed in a sensor block 23, respectively, so that the sensor block 23 is attached while being movable in the up-down direction relative to the tube guide 21 as shown in FIG. 3.

A first ultrasonic element 24 is attached to the lower surface of the sensor block 23. The lower surface of the first ultrasonic element 24 is retractably movable in the tube groove 18 of the tube guide 21 via a through hole 21a drilled in the upper surface of the U-like cross section part 21A of the tube guide 21.

Meanwhile, a second ultrasonic element 25 is attached to the cover 11B as shown in FIG. 2. When the cover 11B is closed, the first ultrasonic element 24 and the second ultrasonic element 25 face with each other with the tube 19 fitted with the tube groove 18 intervening therebetween.

In the example, the sensor block 23 constitutes a first support member for supporting the first ultrasonic element 24. The cover 11B constitutes a second support member for supporting the second ultrasonic element 25. As is described below, in the state where the first ultrasonic element 24 and the second ultrasonic element 25 face with each other, the sensor block 23 having the first ultrasonic element 24 attached thereto is moved reciprocably (moved up and down) along the guide pins 22 to allow adjustment of the distance between the first ultrasonic element 24 and the second ultrasonic element 25.

The ultrasonic waves transmitted from one of the first ultrasonic element 24 and the second ultrasonic element 25 are received by the other ultrasonic element so that existence of bubbles in the blood flowing through the tube 19 can be detected as is well known in the art.

Referring to FIG. 3, the bubble detector 1 includes a gate-type slide block 28 for moving the sensor block 23 up and down along the guide pins 22. Each inner surface of both legs 28A of the gate-type slide block 28 is in sliding contact with the facing surface of the U-like cross section part 21A of the tube guide 21. Each lower surface of both legs 28A is in sliding contact with the surface of the base part 21B of the tube guide 21. The slide block 28 is thus guided by the respective sliding contact surfaces to be reciprocably movable in the axial direction of the tube 19, that is, the longitudinal direction of the U-like cross section part 21A.

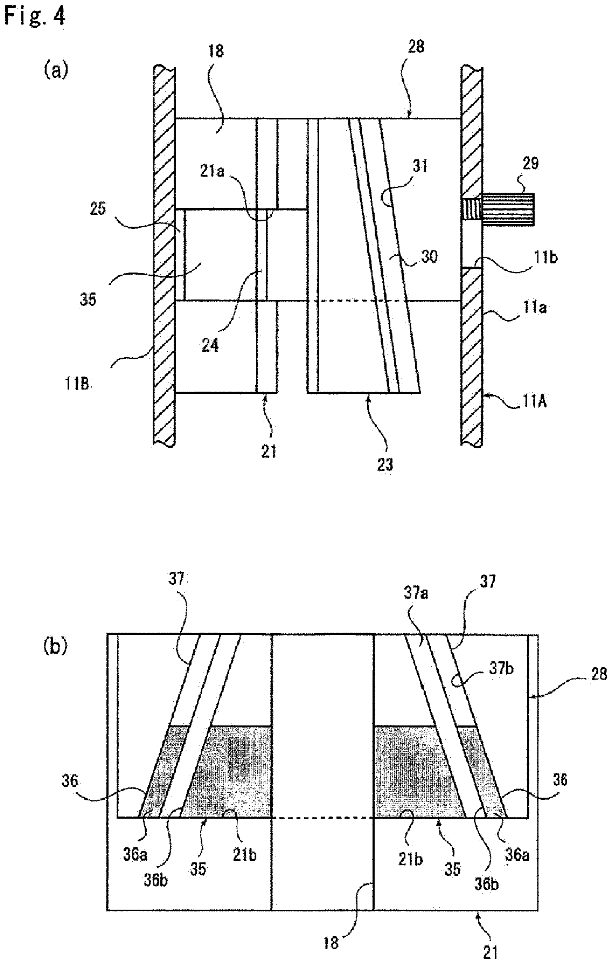

As shown in FIG. 4(a), the upper surface of the slide block 28 is in sliding contact with the inner surface of a wall 11a constituting the main body 11A of the detection box 11. This prevents the slide block 28 from being separated from the tube guide 21 upward in FIG. 3. When the cover 11B is closed, the wall 11a faces the cover 11B parallel to each other. When the detection box 11 is fitted with the recess part 12a of the bracket 12, the wall 11a is positioned on the inner surface side of the recess part 12a.

As shown in FIG. 3 and FIG. 4(a), two adjustment screws 29 are threaded in the respective ends of the slide block 28. Each of the adjustment screws 29 penetrates through a long hole 11b formed in the wall 11a of the main body 11A to protrude to the outside. The long hole 11b is formed in the longitudinal direction of the tube 19. When each of the adjustment screws 29 is tightened at an appropriate position determined by longitudinally moving the slide block 28, the slide block 28 can be fixed to the wall 11a of the main body 11A, that is, the tube guide 21.

As shown in FIG. 3, the sensor block 23 has an upper surface inclined along a longitudinal direction of the tube 19. A first engagement portion 30 with a dovetailing cross section is disposed on the inclined surface. Meanwhile, the lower surface of the gate-type slide block 28, which is positioned between the two legs 28A, is inclined along the inclined surface. A first guide portion 31 with a dovetailing cross section, which is engaged with the first engagement portion 30, is disposed in the inclined surface.

The first engagement portion 30 and the first guide portion 31 have the dovetailing cross sections, by which those portions are engaged to be slidable relative to each other along the longitudinal direction of the inclined surface. However, they cannot be disengaged in the up-down direction.

When the slide block 28 is reciprocably moved in the longitudinal direction of the tube 19 relative to the fixed tube guide 21, each inclination of the first engagement portion 30 and the first guide portion 31 allows the sensor block 23 to move up and down along the guide pins 22.

The first ultrasonic element 24 disposed on the sensor block 23 is moved to approach or separate from the second ultrasonic element 25 disposed on the cover 11B so that the distance between those elements is adjustable.

In this example, a first distance adjuster for adjusting the distance between the first ultrasonic element 24 and the second ultrasonic element 25 is constituted by the slide block 28, the first engagement portion 30, the first guide portion 31, the guide pins 22 and the engagement holes 23a.

The bubble detector 1 includes a pair of side blocks 35 which face with each other with the tube 19 intervening therebetween, and are disposed in a lateral direction intersecting the direction in which the first ultrasonic element 24 and the second ultrasonic element 25 are arranged to face with each other vertically with the tube 19 intervening therebetween. The distance between the side blocks 35 can be adjusted in accordance with the diameter of the tube 19.

Each of the left and right base parts 21B of the tube guide 21 includes a guide hole 21b to be engaged with the corresponding side block 35. Each of the side blocks 35 is engaged with each of the guide holes 21b, and reciprocably movable diametrally with respect to the tube 19 while being protrudable to the inside of the tube groove 18.

Each of the side blocks 35 has a second engagement portion 36 at an outer end, which is inclined with respect to the longitudinal direction of the tube 19. Each second engagement portion 36 includes an outer protrusion 36a and an inner engagement groove 36b. The engagement groove 36b has its depth corresponding to the surface level of the base part 21B.

Meanwhile, each of the legs 28A of the gate-type slide block 28 has a second guide portion 37 formed in the lower surface on the inner side, which is engaged with the second engagement portion 36. Each of the second guide portions 37 is inclined along the inclination of the second engagement portion 36, and has a protrusion 37a to be engaged with the engagement groove 36b of the second engagement portion 36, and an engagement groove 37b to be engaged with the protrusion 36a of the second engagement portion 36 (see FIG. 4(b)).

When the slide block 28 is reciprocably moved relative to the tube guide 21 in the longitudinal direction of the tube 19, the inclination of the second engagement portion 36 and the second guide portion 37 serves to bring the side blocks 35 into approach state or separation state. It is therefore possible to adjust the distance between those side blocks.

At this time, the side blocks 35 move to approach or separate from the center line of the tube groove 18. This makes it possible to hold the tube 19 fitted with the tube groove 18 constantly in the center of the tube groove 18.

In the embodiment, the slide block 28, the second engagement portions 36, and the second guide portions 37 constitute a second distance adjuster for adjusting the distance between the side blocks 35.

As shown in FIG. 1, an arterial-side clamp 41 is disposed on the more upstream side than the arterial-side bubble sensor 1. A venous-side clamp 42 is disposed on the more downstream side than the venous-side bubble sensor 1.

Those clamps 41, 42 are not provided for the detection box 11 but for the blood purifier. Although not illustrated, upon attachment of the detection box 11 to the bracket 12 of the blood purifier, the respective top ends of the clamps 41, 42 are positioned while protruding into enlarged diameter portions 18a of the tube grooves 18 formed in the main body 11A of the detection box 11.

In other words, the detection box 11 can be removed from the bracket 12 while the clamps 41, 42 are left on the blood purifier side. Meanwhile, in the state where the detection box 11 is attached to the bracket 12, the respective top ends of the clamps 41, 42 are positioned in the enlarged diameter portions 18a of the tube grooves 18. Accordingly, the tube 19 may be engaged with the top ends of the clamps 41, 42 upon fitting of the tube 19 with the inside of the tube groove 18. Operation of the clamps 41, 42 in this state serves to press the tube 19 so that blood circulation can be blocked. In this example, the detection box 11 is removed from the bracket 12 while the clamps 41, 42 are left on the blood purifier side. It is possible to make the detection box 11 and the bracket 12 in an integrated manner, or make the detection box 11, the bracket 12, and a clamp box having the clamps 41, 42 in an integrated manner. Such an integrated configuration may be removed from the front panel of the blood purifier so as to allow adjustment of the distance between the first ultrasonic element 24 and the second ultrasonic element 25, and the distance between the side blocks 35.

The above-described structure as shown in FIG. 4 indicates the initial state where the slide block 28 is at the uppermost position with respect to the tube guide 21 in the drawing. In the initial state, the first ultrasonic element 24 provided to the sensor block 23 is separated from the second ultrasonic element 25 provided to the cover 11B at the farthest position (see FIG. 4(a)). Simultaneously, the paired side blocks 35 are separated from each other at the farthest positions (see FIG. 4(b)).

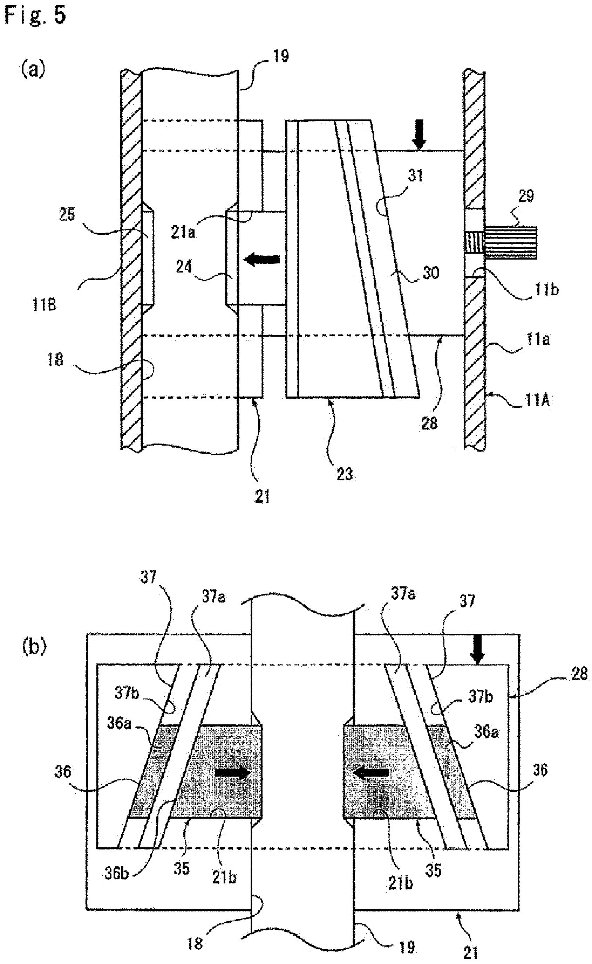

In the case of using a large-diameter tube 19 from the initial state as shown in FIG. 4, the detection box 11 is removed from the bracket 12, and then the adjustment screws 29 are loosened to move the slide block 28 downward as shown in FIG. 5(a).

The first guide portion 31 of the slide block 28 is thus engaged with the first engagement portion 30 of the sensor block 23 to allow the sensor block 23 and the first ultrasonic element 24 attached thereto to be pushed leftward as shown in FIG. 5(a). This makes it possible to reduce the distance between the first ultrasonic element 24 and the second ultrasonic element 25 when the cover 11B is closed.

At the same time, when the slide block 28 is moved downward as shown in FIG. 5(a), each of the second guide portions 37 of the slide block 28 is engaged with each of the second engagement portions 36 of the side blocks 35 so that the distance between the paired side blocks 35 can be reduced.

At a side of the long hole 11b through which the adjustment screw 29 penetrates, a non-illustrated appropriate mark is placed at a position adapted to the diameter of the large-diameter tube 19 to be used. The adjustment screws 29 are moved to the marked position and tightened so that the slide block 28 is fixed to the wall 11a of the detection box 11. This makes it possible to fix the first ultrasonic element 24 and the paired side blocks 35 at positions optimumly adapted to the large-diameter tube 19. Besides placement of the mark, it is possible to provide one end of the long hole at a position adapted to the large-diameter tube 19, and the other end at a position adapted to the small-diameter tube 19, and it is also possible to form holes by the number corresponding to the number of tube sizes, or to bring a jig corresponding to the tube diameter into abutment for adjustment, all of which are by way of example only.

In this way, the position of the slide block 28 is adjusted to the optimum one adapted to the large-diameter tube 19. The detection box 11 is then fitted with the recess part 12a of the bracket 12 of the blood purifier.

Opening the cover 11B of the detection box 11 in the above-described state allows the tube 19 constituting the arterial-side circuit 2A, and the tube 19 constituting the venous-side blood circuit 2B in the blood circuit 2 to be fitted with the respective tube grooves 18. The cover 11B can be closed after fitting of the respective tubes 19 with the tube grooves 18.

In this state, since the distance between the first ultrasonic element 24 and the second ultrasonic element 25, and the distance between the paired side blocks 35 are adjusted to take positions optimumly adapted to the large-diameter tube 19, respectively, the large-diameter tube 19 is held and pressed rectangularly in the up-down direction and left-right direction with respect to the cross section.

Consequently, the first ultrasonic element 24 and the second ultrasonic element 25 are brought into tight contact with the outer circumferential surface of the tube 19 appropriately, resulting in secure bubble detection. At the same time, as the tube 19 is rectangularly pressed, a larger flow passage area is secured compared with the case where the tube 19 is elliptically pressed only by the first ultrasonic element 24 and the second ultrasonic element 25. This makes it possible to prevent the risk of failing to secure required blood quantity caused by the reduced flow passage area.

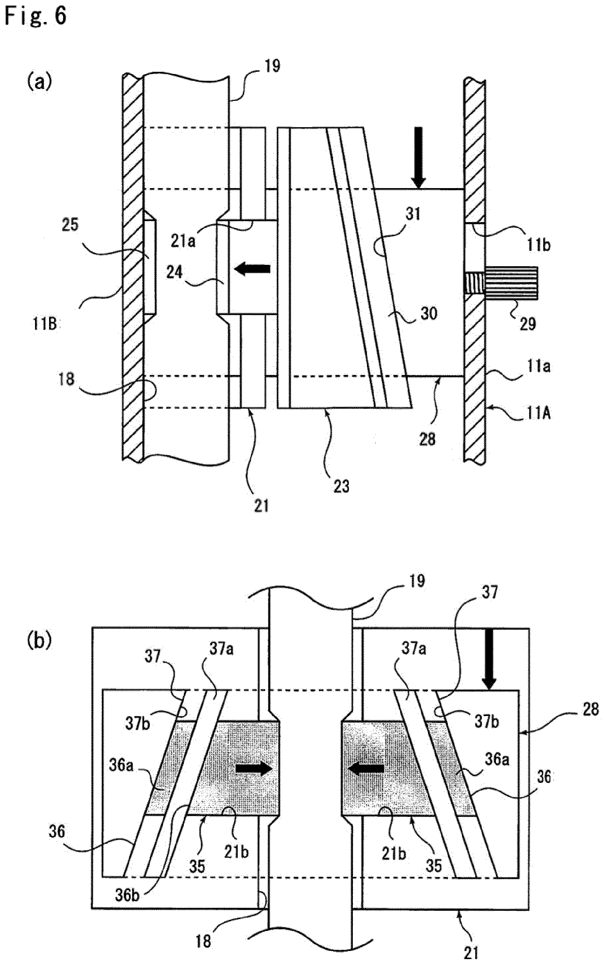

If the use of the small-diameter tube 19 as indicated by FIG. 6 is required from the initial state or the state where the large-diameter tube 19 has been used, the adjustment screws 29 can be loosened to move the slide block 28 downward as shown in FIG. 6(a) similarly to the case as described above. The adjustment screws 29 then can be moved to a non-illustrated marked position placed at the side of the long hole 11b, which is adapted to the small-diameter tube 19, and then tightened.

As shown in FIG. 6(a), this allows the first ultrasonic element 24 to be further pushed leftward, and the distance between the paired side blocks 35 to be further reduced as shown in FIG. 6(b). This makes it possible to bring the first ultrasonic element 24 and the paired side blocks 35 to the optimum positions adapted to the small-diameter tube 19, respectively.

Also in this case, the first ultrasonic element 24 and the second ultrasonic element 25 can also be brought into tight contact with the outer circumferential surface of the small-diameter tube 19 appropriately, resulting in secure bubble detection. Furthermore, as the small-diameter tube 19 can be rectangularly pressed, it is possible to suppress reduction in the flow passage area of the tube 19.

In the example, the slide block 28 is made slidable to allow adjustment of the distance between the first ultrasonic element 24 and the second ultrasonic element 25, and the distance between the paired side blocks 35 simultaneously. Moreover, those adjustments may be performed individually. More specifically, although not illustrated, the sensor block 23 is operated in an interconnected manner with, for example, a position adjustment screw as the first distance adjuster for reciprocably moving the position of the sensor block 23. The position adjustment screw allows adjustment of the distance between the first ultrasonic element 24 and the second ultrasonic element 25 by moving the position of the sensor block 23 reciprocably. Meanwhile, at least one of the paired side blocks 35 is also operated in an interconnected manner with a position adjustment screw as the second distance adjuster for reciprocably moving the position of at least one of the paired side blocks 35. The position adjustment screw allows adjustment of the distance between the side blocks 35 by moving the position of at least one of the side blocks 23 reciprocably.

**[Reference Signs List]**

| | | | |
|---|---|---|---|
| 1 | bubble detector | 11 | detection box |
| 11A | main body | 11B | cover (second support member) |
| 18 | tube groove | 19 | tube |
| 21 | tube guide | 23 | sensor block (first support |
| member ) | | | |
| 24 | first ultrasonic element | 25 | second ultrasonic |
| element | | | |
| 28 | slide block | 29 | adjustment screw |
| 30 | first engagement portion | 31 | first guide portion |
| 35 | side block | 36 | second engagement portion |
| 37 | second guide portion | | |

## Claims

1. A bubble detector (1) comprising a first ultrasonic element (24) which is disposed on a first support member (23), a second ultrasonic element (25) which is disposed on a second support member (11B) to face the first ultrasonic element (24) with a tube (19) intervening therebetween, and a first distance adjuster for adjusting a distance between the first support member (23) and the second support member (11B) to adjust a distance between the first ultrasonic element (24) and the second ultrasonic element (25) in accordance with a diameter of the tube (19), the bubble detector (1) allowing one of the first (24) and the second (25) ultrasonic elements to transmit ultrasonic waves, and the other to receive the ultrasonic waves for detecting existence of bubbles in a liquid flowing through the tube (19), wherein
a pair of side guides (35) which face each other with the tube (19) intervening therebetween are disposed in a radial direction of the tube (19) intersecting a direction in which the first (24) and the second (25) ultrasonic elements are arranged to face each other with the tube (19) intervening therebetween, and a second distance adjuster is provided for adjusting a distance between the pair of side guides (35) in accordance with the diameter of the tube (19); wherein the first distance adjuster and the second distance adjuster are operable to adjust the distance between the first ultrasonic element (24) and the second ultrasonic element (25) and the distance between the pair of side guides (35) simultaneously, such that the first ultrasonic element (24) and the second ultrasonic element (25) are brought into tight contact with the tube (19) as the tube (19) is held and pressed rectangularly.

2. The bubble detector (1) according to claim 1, further comprising a tube guide (21) having a tube groove (18) with which the tube (19) is detachably fitted, and a cover openably provided to the tube guide (21) to allow the tube (19) to be detachably fitted with the tube groove (18) in an open state, and to cover the tube (19) fitted with the tube groove (18) in a closed state, wherein
the second support member (11B) is provided for the cover, and the first support member (23) is provided movably relative to the tube guide (21) in the radial direction of the tube (19) fitted with the tube groove (18), and the first distance adjuster adjusts the distance between the first ultrasonic element (24) and the second ultrasonic element (25) by moving the first support member (11B) in the radial direction of the tube (19), and
the pair of side guides (35) are provided movably relative to the tube guide (21) in the radial direction of the tube (19) fitted with the tube groove (18), and the second distance adjuster moves each of the side guides (35) in the radial direction of the tube (19) to adjust the distance between the side guides (35) in accordance with the diameter of the tube (19).

3. The bubble detector (1) according to claim 2, wherein the first distance adjuster includes a slide block (28) movable along a longitudinal direction of the tube groove (18), a first guide portion (31) that is provided for the slide block, and inclined with respect to the longitudinal direction of the tube groove (18), and a first engagement portion (30) which is disposed on the first support member (23) and engaged with the first guide portion (31) of the slide block (28), and wherein when the slide block (28) is moved along the longitudinal direction of the tube groove (18), the first support member (23) moves reciprocally in the radial direction of the tube (19) via the first guide portion (31) and the first engagement portion (30).

4. The bubble detector (1) according to claim 3, wherein the second distance adjuster includes a pair of second guide portions (37) which are provided for the slide block (28), and inclined with respect to the longitudinal direction of the tube groove (18), and second engagement portions (36) provided for the pair of side guides (35) to be engaged with the second guide portions (37), respectively, and wherein when the slide block (28) is moved along the longitudinal direction of the tube groove (18) the pair of side guides (35) move reciprocally in the radial direction of the tube via the second guide portions (37) and the second engagement portions (36).

## Patentansprüche

1. Bläschendetektor (1), umfassend ein erstes Ultraschallelement (24), das auf einem ersten Halteelement (23) angeordnet ist, ein zweites Ultraschallelement (25), das auf einem zweiten Halteelement (11B) angeordnet ist, um dem ersten Ultraschallelement (24) mit einem dazwischen angeordneten Rohr (19) zugewandt zu sein, und ein erstes Abstandeinstellelement zum Einstellen eines Abstands zwischen dem ersten Halteelement (23) und dem zweiten Halteelement (11B), um einen Abstand zwischen dem ersten Ultraschallelement (24) und dem zweiten Ultraschallelement (25) gemäß einem Durchmesser des Rohres (19) einzustellen, wobei der Bläschendetektor (1) es einem aus dem ersten (24) und dem zweiten (25) Ultraschallelement erlaubt, Ultraschallwellen auszusenden, und es dem anderen erlaubt, die Ultraschallwellen zum Detektieren des Vorliegens von Bläschen in einer durch das Rohr (19) strömenden Flüssigkeit zu empfangen, wobei
ein Paar von Seitenführungen (35), die mit dem dazwischen angeordneten Rohr (19) einander zugewandt sind, in radialer Richtung des Rohres (19), die eine Richtung kreuzt, in der das erste (24) und das zweite (25) Ultraschallelement angeordnet sind, um mit dem dazwischen angeordneten Rohr (19) einander zugewandt zu sein, und ein zweites Abstandeinstellelement zum Einstellen eines Abstands zwischen dem Paar von Seitenführungen (35) gemäß dem Durchmesser des Rohres (19) bereitgestellt ist; wobei das erste Abstandeinstellelement und das zweite Abstandeinstellelement betätigbar sind, um den Abstand zwischen dem ersten Ultraschallelement (24) und dem zweiten Ultraschallelement (25) sowie den Abstand zwischen dem Paar von Seitenführungen (35) gleichzeitig einzustellen, sodass das erste Ultraschallelement (24) und das zweite Ultraschallelement (25) in engen Kontakt mit dem Rohr (19) gebracht werden, wobei das Rohr (19) gehalten und rechtwinklig gedrückt wird.

2. Bläschendetektor (1) nach Anspruch 1, ferner umfassend eine Rohrführung (21) mit einer Rohrvertiefung (18), in der das Rohr (19) lösbar montiert ist, und einer Abdeckung, die öffenbar für die Rohrführung (21) bereitgestellt ist, um es dem Rohr (19) zu ermöglichen, in einem offenen Zustand lösbar in der Rohrvertiefung (18) montiert zu sein und in einem geschlossenen Zustand das in der Rohrvertiefung (18) montierte Rohr (19) abzudecken, wobei
das zweite Halteelement (11B) für die Abdeckung bereitgestellt ist und das erste Halteelement (23) relativ zur Rohrführung (21) in radialer Richtung des in der Rohrvertiefung (18) montierten Rohres (19) bewegbar bereitgestellt ist, und wobei das erste Abstandeinstellelement den Abstand zwischen dem ersten Ultraschallelement (24) und dem zweiten Ultraschallelement (25) durch Bewegen des ersten Halteelements (11B) in radialer Richtung des Rohrs (19) einstellt, und
das Paar von Seitenführungen (35) relativ zur Rohrführung (21) in radialer Richtung des in der Rohrvertiefung (18) montierten Rohres (19) bewegbar bereitgestellt ist und das zweite Abstandeinstellelement jede der Seitenführungen (35) in radialer Richtung des Rohres (19) bewegt, um den Abstand zwischen den Seitenführungen (35) gemäß dem Durchmesser des Rohres (19) einzustellen.

3. Bläschendetektor (1) nach Anspruch 2, wobei das erste Abstandeinstellelement einen Gleitblock (28), der entlang einer Längsrichtung der Rohrvertiefung (18) bewegbar ist, einen ersten Führungsabschnitt (31), der für den Gleitblock bereitgestellt und in Bezug auf die Längsrichtung der Rohrvertiefung (18) geneigt ist, und einen ersten Eingriffsabschnitt (30) umfasst, der auf dem ersten Halteelement (23) angeordnet und mit dem ersten Führungsabschnitt (31) des Gleitblocks (28) in Eingriff steht, und wobei, wenn der Gleitblock (28) entlang der Längsrichtung der Rohrvertiefung (18) bewegt wird, das erste Halteelement (23) sich in radialer Richtung des Rohres (19) über den ersten Führungsabschnitt (31) und den ersten Eingriffsabschnitt (30) hin- und herbewegt.

4. Bläschendetektor (1) nach Anspruch 3, wobei das zweite Abstandseinstellelement ein Paar von zweiten Führungsabschnitten (37), das für den Gleitblock (28) bereitgestellt und in Bezug auf die Längsrichtung der Rohrvertiefung (18) geneigt ist, und zweite Eingriffsabschnitte (36) umfasst, die für das Paar von Seitenführungen (35) bereitgestellt sind, um jeweils mit den zweiten Führungsabschnitten (37) in Eingriff zu gelangen, und wobei, wenn der Gleitblock (28) entlang der Längsrichtung der Rohrvertiefung (18) bewegt wird, das Paar von Seitenführungen (35) sich in radialer Richtung des Rohres über die zweiten Führungsabschnitte (37) und die zweiten Eingriffsabschnitte (36) hin- und herbewegt.

## Revendications

1. Détecteur de bulles (1) comprenant un premier élément ultrasonore (24) qui est disposé sur un premier élément de support (23), un second élément ultrasonore (25) qui est disposé sur un second élément de support (11B) pour faire face au premier élément ultrasonore (24) avec un tube (19) intervenant entre eux, et un premier dispositif d'ajustement de distance pour ajuster une distance entre le premier élément de support (23) et le second élément de support (11B) afin d'ajuster une distance entre le premier élément ultrasonore (24) et le second élément ultrasonore (25) en fonction d'un diamètre du tube (19), le détecteur de bulles (1) permettant à un certain des premier (24) et second (25) éléments ultrasonores de transmettre des ondes ultrasonores, et à l'autre de recevoir les ondes ultrasonores pour détecter l'existence de bulles dans un liquide s'écoulant à travers le tube (19), dans lequel
une paire de guides latéraux (35) qui se font face avec le tube (19) intervenant entre eux sont disposés dans une direction radiale du tube (19) croisant une direction dans laquelle les premier (24) et second (25) éléments ultrasonores sont agencés pour se faire face mutuellement avec le tube (19) intervenant entre eux, et un second dispositif d'ajustement de distance est prévu pour ajuster une distance entre la paire de guides latéraux (35) en fonction du diamètre du tube (19) ; dans lequel le premier dispositif d'ajustement de distance et le second dispositif d'ajustement de distance peuvent fonctionner pour ajuster la distance entre le premier élément ultrasonore (24) et le second élément ultrasonore (25) et la distance entre la paire de guides latéraux (35) simultanément, de sorte que le premier élément ultrasonore (24) et le second élément ultrasonore (25) sont amenés en contact étroit avec le tube (19) lorsque le tube (19) est en champ et pressé rectangulairement.

2. Détecteur de bulles (1) selon la revendication 1, comprenant en outre un guide de tube (21) présentant une rainure de tube (18) avec laquelle le tube (19) est raccordé de manière amovible, et un couvercle prévu de manière à pouvoir être ouvert sur le guide de tube (21) afin de permettre au tube (19) d'être raccordé de manière amovible avec la rainure de tube (18) dans un état ouvert, et pour couvrir le tube (19) raccordé avec la rainure de tube (18) dans un état fermé, dans lequel
le second élément de support (11B) est prévu pour le couvercle, et le premier élément de support (23) est prévu mobile par rapport au guide de tube (21) dans la direction radiale du tube (19) raccordé avec la rainure de tube (18), et le premier dispositif d'ajustement de distance ajuste la distance entre le premier élément ultrasonore (24) et le second élément ultrasonore (25) en déplaçant le premier élément de support (11B) dans la direction radiale du tube (19), et
la paire de guides latéraux (35) est fournie de manière mobile par rapport au guide de tube (21) dans la direction radiale du tube (19) raccordé avec la rainure de tube (18), et le second dispositif d'ajustement de distance déplace chacun des guides latéraux (35) dans la direction radiale du tube (19) pour ajuster la distance entre les guides latéraux (35) en fonction du diamètre du tube (19).

3. Détecteur de bulles (1) selon la revendication 2,
dans lequel le premier dispositif d'ajustement de distance inclut un bloc coulissant (28) mobile le long d'une direction longitudinale de la rainure de tube (18), une première partie de guidage (31) qui est prévue pour le bloc coulissant, et inclinée par rapport à la direction longitudinale de la rainure de tube (18), et une première partie de mise en prise (30) qui est disposée sur le premier élément de support (23) et mise en prise avec la première partie de guidage (31) du bloc coulissant (28), et dans lequel lorsque le bloc coulissant (28) est déplacé le long de la direction longitudinale de la rainure de tube (18), le premier élément de support (23) se déplace en va-et-vient dans la direction radiale du tube (19) via la première partie de guidage (31) et la première partie de mise en prise (30).

4. Détecteur de bulles (1) selon la revendication 3,
dans lequel le second dispositif d'ajustement de distance inclut une paire de secondes parties de guidage (37) qui sont prévues pour le bloc coulissant (28), et inclinées par rapport à la direction longitudinale de la rainure de tube (18), et des secondes parties de mise en prise (36) prévues pour que la paire de guides latéraux (35) soit mise en prise avec les secondes parties de guidage (37), respectivement, et dans lequel lorsque le bloc coulissant (28) est déplacé le long de la direction longitudinale de la rainure de tube (18), la paire de guides latéraux (35) se déplace en va-et-vient dans la direction radiale du tube via les secondes parties de guidage (37) et les secondes parties de mise en prise (36).
